# EUROPEAN PATENT SPECIFICATION

(11) **EP 1 904 490 B1**
(45) Date of publication and mention of the grant of the patent: **29.09.2010**
(21) Application number: 06755779.3
(22) Date of filing: 17.07.2006
(51) Int. Cl.: C07D 413/14, A61K 31/497, A61P 35/04

(54) **ETHANOLAMINE SALT OF N- (3-METHOXY-5-METHYLPYRAZIN-2YL) -2- (4- [1 , 3 , 4-0XADIAZ0LE-2-YL]PHENYL) PYRIDINE-3- SULPHONAMIDE**
ETHANOLAMINSALZ VON N-(METHOXY-5-METHYLPYRAZIN-2-YL)-2-(4-[1,3,4-OXADIAZOL-2-YL]PHENYL)PYRIDIN-3-SULFONAMID
SEL ETHANOLAMINE DE N-(3-METHOXY-5-METHYLPYRAZIN-2-YL)-2-(4-[1,3,4-OXADIAZOLE-2-YL]PHENYL)PYRIDINE-3-SULFAMIDE

(30) Priority: 19.07.2005 GB 0514743
(43) Date of publication of application: 02.04.2008
(73) Proprietor: AstraZeneca AB, 151 85 Södertälje (SE)
(72) Inventor: GOOD, Catherine, Macclesfield Cheshire SK10 4TG (GB); HOGAN, Phillip John, Macclesfield Cheshire SK10 2NA (GB); MONTGOMERY, Frank, Macclesfield Cheshire SK10 4TG (GB)
(74) Representative: Nelson, Michael Andrew
(86) International application number: PCT/GB2006/002654
(87) International publication number: WO 2007/010235

(56) References cited:
- WO-A-96/40681
- WO-A-2004/018044
- CHEONG H-A ET AL: "Enhanced percutaneous absoption of piroxicam via salt formation with ethanolamines" PHARMACEUTICAL RESEARCH 01 SEP 2002 UNITED STATES, vol. 19, no. 9, 1 September 2002 (2002-09-01), pages 1375-1380, XP009071897 ISSN: 0724-8741

## Description

The present application refers to a novel salt of *N*-(3-methoxy-5-methylpyrazin-2-yl)-2-(4-[1,3,4-oxadiazol-2-yl]phenyl)pyridine-3-sulphonamide (hereafter "Compound **(I)).** More specifically the invention relates to the ethanolamine salt of Compound **(I)** (hereafter "Compound **(I)** ethanolamine salt), and to pharmaceutical compositions containing it. The invention further relates to the use of Compound **(I)** ethanolamine salt in the manufacture of medicament for use in treating cancer and to methods of treating cancer in a warm blooded animal such as man using this salt. The invention further relates to the use of Compound **(I)** ethanolamine salt in producing Compound **(I)** during manufacture.

Compound **(I)** is an endothelin antagonist. The endothelins are a family of endogenous 21 amino acid peptides comprising three isoforms, endothelin-1 (ET-1), endothelin-2 and endothelin-3. The endothelins are formed by cleavage of the Trp²¹-Val²² bond of their corresponding proendothelins by an endothelin converting enzyme. The endothelins are among the most potent vasoconstrictors known and have a characteristic long duration of action. They exhibit a wide range of other activities including cell proliferation and mitogenesis, extravasation and chemotaxis, and also interact with a number of other vasoactive agents.

The endothelins are released from a range of tissue and cell sources including vascular endothelium, vascular smooth muscle, kidney, liver, uterus, airways, intestine and leukocytes. Release can be stimulated by hypoxia, shear stress, physical injury and a wide range of hormones and cytokines. Elevated endothelin levels have been found in a number of disease states in man including cancers.

Recently, endothelin A receptor antagonists have been identified as potentially of value in the treatment of cancer (Cancer Research, 56, 663-668, February 15th, 1996 and Nature Medicine, Volume 1, Number 9, September 1999, 944-949).

Cancer affects an estimated 10 million people worldwide. This figure includes incidence, prevalence and mortality. More than 4.4 million cancer cases are reported from Asia, including 2.5 million cases from Eastern Asia, which has the highest rate of incidence in the world. By comparison, Europe has 2.8 million cases, North America 1.4 million cases, and Africa 627,000 cases.

In the UK and US, for example, more than one in three people will develop cancer at some point in their life. Cancer mortality in the U.S. is estimated to account for about 600,000 a year, about one in every four deaths, second only to heart disease in percent of all deaths, and second to accidents as a cause of death of children 1-14 years of age. The estimated cancer incidence in the U.S. is now about 1,380,000 new cases annually, exclusive of about 900,000 cases of non-melanotic (basal and squamous cell) skin cancer.

Cancer is also a major cause of morbidity in the UK with nearly 260,000 new cases (excluding non-melanoma skin cancer) registered in 1997. Cancer is a disease that affects mainly older people, with 65% of cases occurring in those over 65. Since the average life expectancy in the UK has almost doubled since the mid nineteenth century, the population at risk of cancer has grown. Death rates from other causes of death, such as heart disease, have fallen in recent years while deaths from cancer have remained relatively stable. The result is that 1 in 3 people will be diagnosed with cancer during their lifetime and 1 in 4 people will die from cancer. In people under the age of 75, deaths from cancer outnumber deaths from diseases of the circulatory system, including ischaemic heart disease and stroke. In 2000, there were 151,200 deaths from cancer. Over one fifth (22 per cent) of these were from lung cancer, and a quarter (26 per cent) from cancers of the large bowel, breast and prostate.

Worldwide, the incidence and mortality rates of certain types of cancer (of stomach, breast, prostate, skin, and so on) have wide geographical differences which are attributed to racial, cultural, and especially environmental influences. There are over 200 different types of cancer but the four major types, lung, breast, prostate and colorectal, account for over half of all cases diagnosed in the UK and US. Prostate cancer is the fourth most common malignancy among men worldwide, with an estimated 400,000 new cases diagnosed annually, accounting for 3.9 percent of all new cancer cases.

Current options for treating cancers include surgical resection, external beam radiation therapy and / or systemic chemotherapy. These are partially successful in some forms of cancer, but are not successful in others. There is a clear need for new therapeutic treatments.

Compound **(I)** is exemplified and described in WO96/40681 as Example 36. WO96/40681 claims the endothelin receptors described therein for the treatment of cardiovascular diseases. The use of Compound **(I)** in the treatment of cancers and pain is described in WO04/018044.

Compound **(I)** has the following structure:

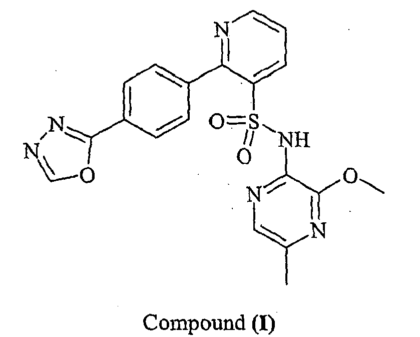

In WO04/018044 an endothelin human receptor binding assay is described. The pIC₅₀ (negative log of the concentration of compound required to displace 50% of the ligand) for Compound **(I)** at the ET_{A} receptor was 8.27 [8.23 - 8.32] (n=4). Compound **(I)** is thus an excellent endothelin antagonist.

WO96/40681 and WO04/018044 disclose, in general terms, certain pharmaceutically acceptable salts of the compounds disclosed therein. Specifically it is stated that suitable pharmaceutically-acceptable salts include, for example, salts with alkali metal (such as sodium, potassium or lithium), alkaline earth metals (such as calcium or magnesium), ammonium salts, and salts with organic bases affording physiologically acceptable cations, such as salts with methylamine, dimethylamine, trimethylamine, piperidine and morpholine. In addition, it was stated that suitable pharmaceutically-acceptable salts include, pharmaceutically-acceptable acid-addition salts with hydrogen halides, sulphuric acid, phosphoric acid and with organic acids such as citric acid, maleic acid, methanesulphonic acid and p-toluenesulphonic acid.

However, nowhere in WO96/40681 or WO04/018044 are specific salts of Compound **(I)** described and nowhere is the potential of forming an ethanolamine salt of Compound **(I)** described.

The present inventors have surprisingly found that Compound **(I)** ethanolamine salt is particularly soluble compared to the free base of Compound **(I)** and other salts. The present inventors measured the intrinsic dissolution rates (IDRs) of Compound **(I)** and those of the sodium salt, ethanolamine salt, ammonium salt and the N-methylpyrrolidinone solvate of the ammonium salt and found that the ammonium salt (as the N-methylpyrrolidinone solvate) was twice as soluble as the free base, the ammonium salt was nearly three time as soluble, but the ethanolamine salt was nearly seventeen times more soluble than the free base. The sodium salt was also more soluble than the free base, but the exact IDR was difficult to measure.

The three salts also had distinctly different stabilities:
- The sodium salt is extremely difficult to convert back to Compound **(I)** without the use of strong acid. It is very stable and 400MHz proton NMR after prolonged storage at ambient shows it to have the same strength and chemical shifts.
- Ammonium Salt: The ammonium salt is very water soluble and the solubility decreases with the addition of industrial methylated spirit (IMS). It can crystallize out of the aqueous IMS reaction liquors. The salt is relatively unstable and will disproportionate back to Compound **(I)** either on ageing at ambient temperature over a prolonged period or on vacuum drying at 50°C overnight;
- Ethanolamine salt: Compound **(I)** ethanolamine salt is extremely soluble in water. It is also very soluble in N-methylpyrrolidinone whereas the ammonium salt is not. The ethanolamine salt also has greater solubility in IMS than the ammonium salt. The salt is stable for 2 days at 50°C assessed by NMR.

More stable forms of a pharmaceutically active compound are preferred for formulation and processing on a commercial scale. This is because the greater the stability of the form used, the lower the risk of it converting to another form during formulation procedures such as compression. This in turn provides greater predictability of the properties of the final formulation, such as dissolution rate of tablets and bioavailability of active ingredient. Furthermore, using a more stable form of an active ingredient allows greater control over the physical properties of the formulation. However, a salt that is too stable is not desirable for large scale manufacture if ultimately a free base is required, because it is difficult to convert back to the free base.

Accordingly, the identification of a salt form of Compound **(I)** that has improved solid state properties is one aspect of the present invention.

According to the present invention there is provided Compound **(I)** ethanolamine salt in substantially crystalline form.

Also provided herein is Compound **(I)** sodium salt in substantially crystalline form.

Also provided herein is Compound **(I)** ammonia salt in substantially crystalline form.

Also provided herein is Compound **(I)** ammonia salt NMP solvate in substantially crystalline form.

According to the present invention there is provided Compound **(I)** ethanolamine salt in a crystalline form characterized in that the compound has an X-ray powder diffraction pattern containing at least peaks with 2-theta values at 8.9°, 10.9° and 18° measured using CuKa radiation.

According to the present invention there is provided Compound **(I)** ethanolamine salt in a crystalline form characterized in that the compound has an X-ray powder diffraction pattern containing at least peaks with 2-theta values at 8.9°, 10.9°, 18°, 25.5°, 15.5°and 21.7° measured using CuKa radiation.

According to the present invention there is provided Compound **(I)** ethanolamine salt in a crystalline form characterized in that the compound has an X-ray powder diffraction pattern containing at least peaks with 2-theta values at 8.9°, 10.9°, 18°, 25.5°, 15.5°, 21.7, 21.2°, 24.1° and 25.9° measured using CuKa radiation.

According to the present invention there is provided Compound **(I)** ethanolamine salt in a crystalline form characterized in that the compound has an X-ray powder diffraction pattern containing at least peaks with 2-theta values at 8.9°, 10.9°, 18.°, 25.5°, 15.5°, 21.7, 21.2°, 24.1°, 25.9°, 13.9° and 35.2° measured using CuKa radiation.

Also provided herein is Compound **(I)** sodium salt in a crystalline form characterized in that the compound has an X-ray powder diffraction pattern containing at least peaks with 2-theta values at 9.3° and 7.1° measured using CuKa radiation.

Also provided herein is Compound **(I)** sodium salt in a crystalline form characterized in that the compound has an X-ray powder diffraction pattern containing at least peaks with 2-theta values at 9.3°, 7.1°, 13.8°, 26.9°, 25.5°, 19.3°, 26.1°, 26.5°, 22.5° and 17.7° measured using CuKa radiation.

Also provided herein is Compound **(I)** ammonia salt in a crystalline form characterized in that the compound has an X-ray powder diffraction pattern containing at least peaks with 2-theta values at 8.4° and 19.6° measured using CuKa radiation.

Also provided herein is Compound **(I)** ammonia salt in a crystalline form characterized in that the compound has an X-ray powder diffraction pattern containing at least peaks with 2-theta values at 8.4°, 19.6°, 24.1°, 8.8°, 13.5°, 25.7°, 12.2°, 25.2°, 17.8° and 18.2° measured using CuKa radiation.

Also provided herein is Compound **(I)** ammonia salt NMP solvate in a crystalline form characterized in that the compound has an X-ray powder diffraction pattern containing at least peaks with 2-theta values at 7.6°, 8.3° and 10.0° measured using CuKa radiation.

Also provided herein is Compound **(I)** ammonia salt NMP solvate in a crystalline form characterized in that the compound has an X-ray powder diffraction pattern containing at least peaks with 2-theta values at 7.6°, 8.3°, 10.0°, 15.6°, 23.1°, 15.2°, 18.0°, 16.6°, 24.6° and 13.0° measured using CuKa radiation.

Where "substantially crystalline form" is referred to, suitably this refers to greater than 50% crystalline. Particularly this refers to greater than 75% crystalline. More particularly this refers to greater than 90% crystalline. Particularly this refers to greater than 99% crystalline.

The present inventors have also found that Compound **(I)** ethanolamine salt is particularly useful when formed *in situ* during the manufacture of Compound **(I).**

The final step of the manufacture of Compound **(I)** is a deprotection step: 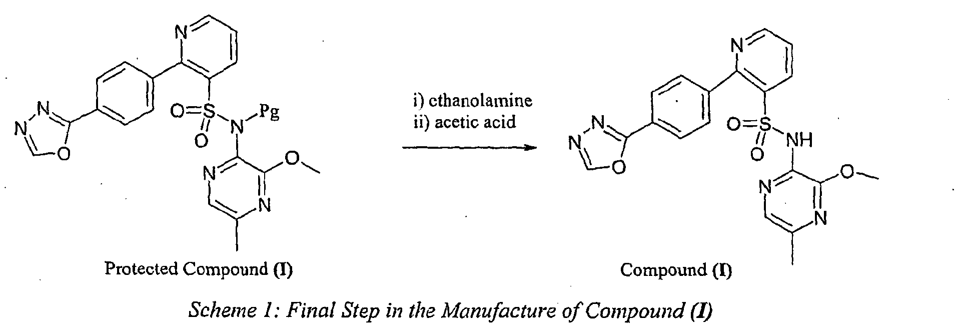 where Pg is a suitable nitrogen protecting group.

A suitable value for Pg is, for example, a C₁₋₆alkoxycarbonyl group, for example a methoxycarbonyl, ethoxycarbonyl, isobutoxycarbonyl or *tert*-butoxycarbonyl group; an arylmethoxycarbonyl group, for example benzyloxycarbonyl. More suitable values for Pg are a methoxycarbonyl, ethoxycarbonyl or isobutoxycarbonyl group. More specifically a value for Pg is isobutoxycarbonyl.

The main impurities present in this reaction from the manufacturing process are: 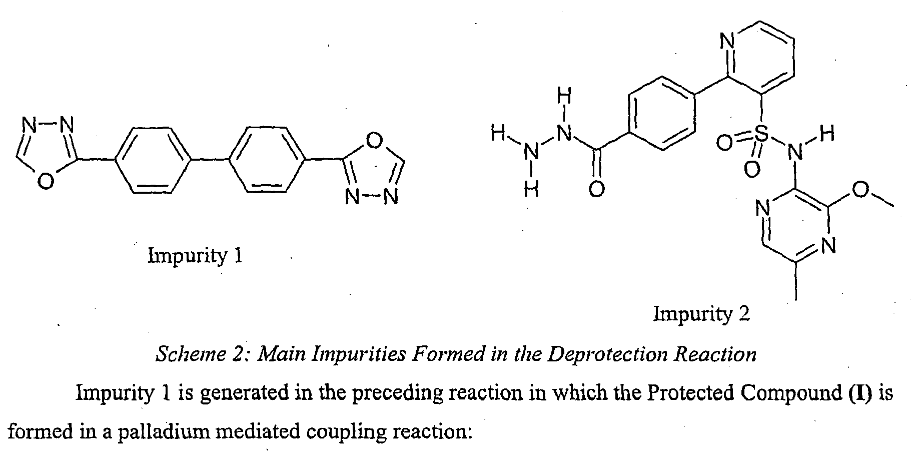 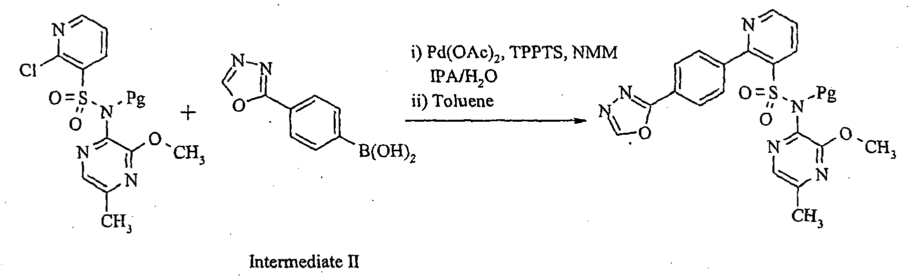 TPPTS = 3,3',3"-phosphinidyne tris(benzenesulphonic acid) trisodium salt;
Pg is a suitable nitrogen protecting group.

This reaction is preferably telescoped into the subsequent deprotection reaction for reasons of process efficiency.

Impurity 2 is formed by decomposition of the oxadiazole ring during the deprotection process.

The present inventors have extensively investigated this deprotection and surprisingly found that using ethanolamine for this deprotection step, thus forming Compound **(I)** ethanolamine salt *in situ,* leads to particular process advantages.

For example, ammonia had been used to effect the above deprotection. The key problem of using ammonia was the poor solubility of the ammonium salt of Compound **(I)** in the reaction medium. A further constraint was the limited stability of Compound **(I)** to ammonia leading to formation of Impurity 2. A solution from which the salt doesn't crystallise, i.e. a stable solution, is desirable at this point for various reasons:
1. It would provide an opportunity to remove Impurity 1. This impurity was present in unacceptable amounts in all the batches from some manufacturing campaigns. The impurity is not significantly reduced in the final purification of Compound **(I)** so must be reduced during the deprotection step. As the impurity is not particularly soluble it is present as a precipitate and therefore a stable solution of a salt of Compound **(I)** would provide an opportunity to remove this impurity by filtration.
2. It would allow separation of the organic phase after the deprotection step leaving a low volume aqueous phase which can be added into hot acetic acid to give a much more reliable crystallisation to produce the preferred polymorphic form of Compound **(I).** If the solution to be added to acetic acid contains the organic layer as well, the volume is much larger and the crystallisation less reliable.
3. An absorbent, for example QuadraPure^{™} TU, could be added to the solution to further reduce palladium levels in Compound **(I)** should a lower metals specification limit be required. Palladium levels in the resultant Compound **(I)** must be virtually undetectable.

Following the addition of the salt solution of Compound **(I)** to the acetic acid, typical levels of Impurity 2 in isolated Compound **(I)** from the ammonia process are 0.26-0.07% verses 0.06% in the ethanolamine process. The overall impurity levels from these two procedures are 0.75 - 0.34 % for the ammonia process verses an average of 0.22% for the ethanolamine process.

Due to the high solubility of Compound **(I)** ethanolamine salt in the aqueous IMS phase of the reaction mixture, it can be separated from the organic phase with little or no loss of yield. The aqueous phase containing Compound **(I)** ethanolamine salt can then be charged to hot aqueous acetic acid to crystallise the product. This mode of addition ensures a poorly filtering polymorphic form of Compound **(I)**, referred to as Form 3, is not generated, as Compound **(I)** is soluble in the crystallising medium and can equilibrate to the desired crystalline form, referred to as Form 1 in the Cambridge crystallographic database. [N-(3-Methoxy-5-methylpyrazin-2-yl)-2-[4-(1,3,4-oxadiazol-2-yl)phenyl]pyridine-3-sulfonamide (ZD4054 Form 1). Acta Crystallographica, Section E: Structure Reports Online (2004), E60(10), o1817-o1819].

Compound **(I)** as Form 1 is characterized in that the compound has an X-ray powder diffraction pattern containing at least peaks with 2-theta values at 25.2°, 11.7°, 12.2° and 13.0° measured using CuKa radiation.

Compound **(I)** as Form 1 may be further characterized in that the compound has an X-ray powder diffraction pattern containing at least peaks with 2-theta values at 25.2°, 11.7°, 12.2°, 13.0°, 16.6°, 16.8°, 16.9°, 19.7°, 27.2° and 11.5° measured using CuKa radiation.

Compound **(I)** as Form 3 is characterized in that the compound has an X-ray powder diffraction pattern containing at least peaks with 2-theta values at 5.3°, 11.8°, 10.5° and 15.7° measured using CuKa radiation.

Compound **(I)** as Form 3 may be further characterized in that the compound has an X-ray powder diffraction pattern containing at least peaks with 2-theta values at 5.3°, 11.8°, 10.5°, 15.7°, 21.0°, 23.6°, 13.1°, 25.3°, 26.3° and 19.6° measured using CuKa radiation.

This mode of addition, although feasible with Compound **(I)** ammonium salt, suffered from a number of drawbacks:
1. The crystallised ammonium salt is dispersed through both the organic and the aqueous phases and therefore the phases could not be separated. Thus the whole reaction mass (as opposed to just the aqueous phase for the ethanolamine reaction) had to be added to the acetic acid. More acetic acid was therefore needed and this large volume decreases the efficiency of the process considerably.
2. The presence of the organic phase and impurities contained within it impedes the crystallisation providing poorer crystalline material and impurity removal.

The present inventors investigated alternative bases to use in the deprotection step to find conditions that gave greater solubility of Compound **(I).** Three amine bases containing hydroxyl groups were examined in detail: choline hydroxide, tetrabutylammonium hydroxide and ethanolamine.

Deprotection occurred quickly when either choline hydroxide or tetrabutyl ammonium hydroxide were used. Unfortunately significant decomposition of Compound **(I)** occurred, even at 20°C, leading to formation of large amounts of Impurity 2. This led to yield loss and poor quality product being isolated.

Ethanolamine completed the deprotection in 1 hour (the same time as with ammonia) and provided a stable solution of Compound **(I)** as its ethanolamine salt at end of reaction. The level of formation of Impurity 2 is roughly similar between ammonia and ethanolamine at 40°C (typically 0.3 to 0.6% at end of reaction), but the level of Impurity 2 in the isolated solid is lower in the ethanolamine process as detailed above probably due to the improved crystallisation. However, Protected Compound **(I)** can be deprotected at 20°C with ethanolamine, which reduces the formation of Impurity 2 to virtually undectable levels at end of reaction. This is not possible using the ammonia deprotection as no reaction occurs at this temperature.

To summarize, the advantages of forming Compound **(I)** ethanolamine salt during the deprotection step when compared to other salts tested, are as follows:
- It provides a stable solution, in that the salt does not crystallise from the solution, allowing for an improved isolation procedure.
- There are lower overall impurity levels.
- Fine filtration can be used to filter the solution and reduce the less soluble impurity (Impurity 1).
- There is the potential to add palladium absorbent to the solution to reduce metal contamination.
- There is limited decomposition of Protected Compound **(I)** and Compound **(I)** with ethanolamine.
- There is a controlled formation of the polymorph "Form 1" when the ethanolamine solution is charged to acetic acid.
- There is overall improved process efficiency.

Therefore in one aspect of the invention there is provided the use of Compound **(I)** ethanolamine salt in the preparation of Compound **(I).**

In a further aspect of this invention there is provided a process for the preparation of Compound **(I),** which comprises the use of ethanolamine to deprotect Protected Compound (I) to form Compound **(I).**

In a further aspect of this invention there is provided a process for the preparation of Compound **(I)** which comprises the use of ethanolamine to deprotect N-(isobutoxycarbonyl) N-(3-methoxy-5-methylpyrazin-2-yl)-2-(4-[1,3,4-oxadiazol-2-yl]phenyl) pyridine-3-sulphonamide to form Compound **(I).**

In a further aspect of this invention there is provided the use of Compound **(I)** ethanolamine salt in the manufacture of Compound **(I)** substantially in the form of Form 1.

Substantially in the form of Form 1 means that there is greater than 95% of Form 1 present. In particular there is greater than 96% Form 1. Particularly there is greater than 97% Form 1. In particular there is greater than 98% Form 1. Particularly there is greater than 99% Form 1. In particular there is greater than 99.5% Form 1. Particularly there is greater than 99.8% Form 1.

In a further aspect of this invention there is provided a process for the manufacture of Compound **(I)** substantially in the form of Form 1 which comprises:
(i) the use of ethanolamine to deprotect Protected Compound **(I),** followed by
(ii) the addition of the resulting Compound **(I)** ethanolamine salt to an acid.

In a further aspect of this invention there is provided a process for the manufacture of Compound **(I)** substantially in the form of Form 1 which comprises:
(i) the use of ethanolamine to deprotect Protected Compound **(I),** in the presence of a solvent followed by
(ii) the addition of the resulting Compound **(I)** ethanolamine salt solution to an acid.

Suitably the acid is acetic, propionic, formic, butyric or iso-butyric acid. Particularly the acid is acetic acid. Suitably the acetic acid is 80% acetic 20% water. In another aspect the acetic acid is glacial acetic acid.

Suitably the solvent is an aqueous alcohols, for example methanol, ethanol, iso-propanol, propanol, iso-butanol or butanol, or aqueous NMP, particularly aqueous iso-propanol.

In a further aspect of this invention there is provided a process for the manufacture of Compound **(I)** substantially in the form of Form 1 which comprises:
(i) the use of ethanolamine to deprotect Protected Compound **(I),** followed by
(ii) the addition of the resulting Compound **(I)** ethanolamine salt to acetic acid.

In a further aspect of this invention there is provided a process for the manufacture of Compound **(I)** substantially in the form of Form 1 which comprises:
(i) the use of ethanolamine to deprotect Protected Compound **(I),** in the presence of a solvent followed by
(ii) the addition of the resulting Compound **(I)** ethanolamine salt solution to acetic acid.

In a further aspect of this invention there is provided a process for the manufacture of Compound **(I)** substantially in the form of Form 1 which comprises:
(i) the use of ethanolamine to deprotect N-(isobutoxycarbonyl) N-(3-methoxy-5-methylpyrazin-2-yl)-2-(4-[1,3,4-oxadiazol-2-yl]phenyl) pyridine-3-sulphonamide, followed by
(ii) the addition of the resulting solution of Compound **(1)** ethanolamine salt to an acid.

In a further aspect of this invention there is provided a process for the manufacture of Compound **(I)** substantially in the form of Form 1 which comprises:
(i) the use of ethanolamine to deprotect N-(isobutoxycarbonyl) N-(3-methoxy-5-methylpyrazin-2-yl)-2-(4-[1,3,4-oxadiazol-2-yl]phenyl) pyridine-3-sulphonamide in the presence of aqueous iso-propanol, followed by
(ii) the addition of the resulting solution of Compound **(I)** ethanolamine salt solution to an acid.

In a further aspect of this invention there is provided a process for the manufacture of Compound **(I)** substantially in the form of Form 1 which comprises:
(i) the use of ethanolamine to deprotect N-(isobutoxycarbonyl) N-(3-methoxy-5-methylpyrazin-2-yl)-2-(4-[1,3,4-oxadiazol-2-yl]phenyl) pyridine-3-sulphonamide, followed by
(ii) the addition of the resulting Compound **(I)** ethanolamine salt to acetic acid.

In a further aspect of this invention there is provided a process for the manufacture of Compound **(I)** substantially in the form of Form 1 which comprises:
(i) the use of ethanolamine to deprotect N-(isobutoxycarbonyl) N-(3-methoxy-5-methylpyrazin-2-yl)-2-(4-[1,3,4-oxadiazol-2-yl]phenyl) pyridine-3-sulphonamide in the presence of aqueous iso-propanol, followed by
(ii) the addition of the resulting Compound **(I)** ethanolamine salt solution to acetic acid.

In a further aspect of the present invention Compound **(I)** ethanolamine salt may be used in the treatment or prophylaxis of cancer or pain.

Therefore in a further aspect of the invention there is provided a pharmaceutical composition which comprises Compound **(I)** ethanolamine salt in association with a pharmaceutically acceptable diluent or carrier.

The pharmaceutical compositions may be in a form suitable for oral administration, for example as a tablet or capsule, for parenteral injection (including intravenous, subcutaneous, intramuscular, intravascular or infusion) as a sterile solution, suspension or emulsion, for topical administration as an ointment or cream, for rectal administration or for intranasal administration including a nasal spray formulation. In general the above compositions may be prepared in a conventional manner using conventional excipients and according to methods generally known in the art of formulation technology.

In a further aspect of the invention there is provided the use of Compound **(I)** ethanolamine salt as a medicament.

Therefore according to this aspect of the present invention, there is provided Compound (I) ethanolamine salt, for use in the treatment of cancer in a warm blooded animal such as man.

According to another feature of the present invention, there is provided Compound **(I)** ethanolamine salt, in the manufacture of a medicament for use in the treatment of cancer in a warm blooded animal such as man.

According to a further feature of this aspect of the invention there is provided a pharmaceutical composition which comprises Compound **(I)** ethanolamine salt, in association with a pharmaceutically acceptable diluent or carrier for use in the treatment of cancer in a warm blooded animal such as man.

In another aspect of the invention there is provided a Compound **(I)** ethanolamine salt, for use in the reduction of abnormal proliferation in a cancerous cell or inducing differentiation of a cancerous cell in a warm blooded animal such as man.

In another aspect of the invention there is provided the use of Compound **(I)** ethanolamine salt, in the manufacture of a medicament for use in the reduction of abnormal proliferation in a cancerous cell or inducing differentiation of a cancerous cell in a warm blooded animal such as man.

According to a further feature of this aspect of the invention there is provided a pharmaceutical composition which comprises Compound **(I)** ethanolamine salt, in association with a pharmaceutically acceptable diluent or carrier for use in the reduction of abnormal proliferation in a cancerous cell or inducing differentiation of a cancerous cell in a warm blooded animal such as man.

In another aspect of the invention there is provided the use of Compound **(I)** ethanolamine salt, in inducing apoptosis in a cancerous cell in a warm blooded animal such as man.

In another aspect of the invention there is provided the use of Compound **(I)** ethanolamine salt, in the manufacture of a medicament for use in inducing apoptosis in a cancerous cell in a warm blooded animal such as man.

According to a further feature of this aspect of the invention there is provided a pharmaceutical composition which comprises Compound **(I)** ethanolamine salt, in association with a pharmaceutically acceptable diluent or carrier for use in inducing apoptosis in a cancerous cell in a warm blooded animal such as man.

In another aspect of the invention there is provided a Compound **(I)** ethanolamine salt for use as an anti-angiogenic and vascular targeting agent in blood vessels supplying a cancerous cell in a warm blooded animal such as man.

In another aspect of the invention there is provided the use of Compound **(I)** ethanolamine salt, in the manufacture of a medicament for use as an anti-angiogenic and vascular targeting agent in blood vessels supplying a cancerous cell in a warm blooded animal such as man.

According to a further feature of this aspect of the invention there is provided a pharmaceutical composition which comprises Compound **(I)** ethanolamine salt, in association with a pharmaceutically acceptable diluent or carrier for use as an anti-angiogenic and vascular targeting agent in blood vessels supplying a cancerous cell in a warm blooded animal such as man.

By the term "vascular targeting agent" it is to be understood that the site of action of Compound **(I)** ethanolamine salt would be on the vasculature itself rather than the tumour,

In another aspect of the invention there is provided a Compound **(I)** ethanolamine salt for use as an anti-angiogenic agent in a warm blooded animal such as man.

In another aspect of the invention there is provided the use of Compound **(I)** ethanolamine salt, in the manufacture of a medicament for use as an anti-angiogenic agent in a warm blooded animal such as man.

According to a further feature of this aspect of the invention there is provided a pharmaceutical composition which comprises Compound **(I)** ethanolamine salt, in association with a pharmaceutically acceptable diluent or carrier for use as an anti-angiogenic agent in a warm blooded animal such as man.

In another aspect of the invention there is provided a Compound **(I)** ethanolamine salt, for use as an inhibitor of bone metastases and an inhibitor of invasion in a warm blooded animal such as man.

In another aspect of the invention there is provided the use of Compound **(I)** ethanolamine salt, in the manufacture of a medicament for use as an inhibitor of bone metastases and an inhibitor of invasion in a warm blooded animal such as man.

According to a further feature of this aspect of the invention there is provided a pharmaceutical composition which comprises Compound **(I)** ethanolamine salt, in association with a pharmaceutically acceptable diluent or carrier for use as an inhibitor of bone metastases and an inhibitor of invasion in a warm blooded animal such as man.

In another aspect of the invention there is provided a Compound **(I)** ethanolamine salt, for use as an inhibitor of bone metastases in a warm blooded animal such as man.

In another aspect of the invention there is provided the use of Compound **(I)** ethanolamine salt, in the manufacture of a medicament for use as an inhibitor of bone metastases in a warm blooded animal such as man.

According to a further feature of this aspect of the invention there is provided a pharmaceutical composition which comprises Compound **(I)** ethanolamine salt, in association with a pharmaceutically acceptable diluent or carrier for use as an inhibitor of bone metastases in a warm blooded animal such as man.

In another aspect of the invention there is provided a Compound **(I)** ethanolamine salt, for use in the prevention of bone metastases in a warm blooded animal such as man.

In another aspect of the invention there is provided the use of Compound **(I)** ethanolamine salt, in the manufacture of a medicament for use in the prevention of bone metastases in a warm blooded animal such as man.

According to a further feature of this aspect of the invention there is provided a pharmaceutical composition which comprises Compound **(I)** ethanolamine salt, in association with a pharmaceutically acceptable diluent or carrier for use in the prevention of bone metastases in a warm blooded animal such as man.

In another aspect of the invention there is provided a Compound **(I)** ethanolamine salt, for use in the treatment of bone metastases in a warm blooded animal such as man.

In another aspect of the invention there is provided the use of Compound **(I)** ethanolamine salt, in the manufacture of a medicament for use in the treatment of bone metastases in a warm blooded animal such as man.

According to a further feature of this aspect of the invention there is provided a pharmaceutical composition which comprises Compound **(I)** ethanolamine salt, in association with a pharmaceutically acceptable diluent or carrier for use in the treatment of bone metastases in a warm blooded animal such as man.

Compounds of the invention may be useful in the inhibition, treatment and / or prevention of bone metastases, as described herein, wherein the bone metastases are as a result of renal, thyroid, lung, breast or prostate cancer.

In another aspect of the invention there is provided a Compound **(I)** ethanolamine salt, for use in the prevention or treatment of pain associated with elevated endothelin-1 production in a warm blooded animal such as man.

In another aspect of the invention there is provided the use of Compound **(I)** ethanolamine salt, in the manufacture of a medicament for use in the prevention or treatment of pain associated with elevated endothelin-1 production in a warm blooded animal such as man.

According to a further feature of this aspect of the invention there is provided a pharmaceutical composition which comprises Compound **(I)** ethanolamine salt, in association with a pharmaceutically acceptable diluent or carrier for use in the prevention or treatment of pain associated with elevated endothelin-1 production in a warm blooded animal such as man.

In another aspect of the invention there is provided a Compound **(I)** ethanolamine salt, for use in the prevention or treatment of pain in a warm blooded animal such as man.

In another aspect of the invention there is provided the use of Compound **(I)** ethanolamine salt, in the manufacture of a medicament for use in the prevention or treatment of pain in a warm blooded animal such as man.

In another aspect of the invention there is provided a Compound **(I)** ethanolamine salt, for use in the prevention or treatment of pain associated with stimulation of the ET_{A} receptor in a warm blooded animal such as man.

In another aspect of the invention there is provided the use of Compound **(I)** ethanolamine salt, in the manufacture of a medicament for use in the prevention or treatment of pain associated with stimulation of the ET_{A} receptor in a warm blooded animal such as man.

Where cancer is referred to, particularly it refers to oesophageal cancer, myeloma, hepatocellular, pancreatic, cervical cancer, ewings tumour, neuroblastoma, Kaposis sarcoma, ovarian cancer, breast cancer, colorectal cancer, prostate cancer, bladder cancer, melanoma, lung cancer - non small cell lung cancer (NSCLC), and small cell lung cancer (SCLC) - gastric cancer, head and neck cancer, renal cancer, lymphoma and leukaemia. More particularly it refers to prostate cancer. In addition, more particularly it refers to SCLC, NSCLC, colorectal cancer, ovarian cancer and / or breast cancer. In addition, more particularly it refers to SCLC. In addition, more particularly it refers to NSCLC. In addition, more particularly it refers to colorectal cancer. In addition, more particularly it refers to ovarian cancer. In addition, more particularly it refers to breast cancer. Furthermore, more particularly it refers to bladder cancer, oesophageal cancer, gastric cancer, melanoma, cervical cancer and / or renal cancer. In addition it refers to endometrial, liver, stomach, thyroid, rectal and / or brain cancer. In another aspect of the invention, the cancer is not melanoma. In another embodiment of the invention, particularly the cancer is in a metastatic state, and more particularly the cancer produces metastases to the bone. In a further embodiment of the invention, particularly the cancer is in a metastatic state, and more particularly the cancer produces skin metastases. In a further embodiment of the invention, particularly the cancer is in a metastatic state, and more particularly the cancer produces lymphatic metastases. In a further embodiment of the invention, the cancer is in a non-metastatic state.

It is to be understood that when the cancer is in a metastatic state, that Compound **(I)** ethanolamine salt acts at both the primary tumour site and the metastases by prevention, treatment and inhibition of metastases.

In one aspect of the invention, where pain is referred to, this is pain associated with raised endothelin-1 levels. In another aspect of the invention this is pain associated with stimulation of the ET_{A} receptor resulting from situations where ET_{B} down-regulation has occurred leading to abnormal ET_{A} stimulation and/or elevation of endothelin-1 levels. Particularly this is pain associated with cancer. More particularly it is pain associated with prostate cancer.

According to a further feature of this aspect of the invention there is provided a pharmaceutical composition which comprises Compound **(I)** ethanolamine salt, in association with a pharmaceutically acceptable diluent or carrier for use in the prevention or treatment of pain associated with stimulation of the ET_{A} receptor in a warm blooded animal such as man.

Additionally, Compound **(I)** ethanolamine salt is expected to be useful in the treatment and/or prophylaxis of pain of different origins and causes, including acute as well as chronic pain states. Examples are pain caused by chemical, mechanical, radiation (including sunburn), thermal (including burns), infectious or inflammatory tissue trauma or cancer, postoperative pain, post-partum pain, the pain associated with joint conditions (such as rheumatoid arthritis and osteoarthritis), pain associated with dental conditions (such as dental caries and gingivitis), myofascial and low back pain, pain associated with bone disorders (such as osteoporosis, hypercalcaemia of malignancy and Paget's disease) and the pain associated with sports injuries and sprains.

Also neuropathic pain conditions of central or peripheral origin could be treated or prevented with Compound **(I)** ethanolamine salt. Examples of these pain conditions are pain associated with trigeminal neuralgia, pain associated with postherpetic neuralgia (PHN), pain associated with diabetic mono/poly neuropathy, pain associated with nerve trauma, pain associated with spinal cord injury, pain associated with central post stroke, pain associated with multiple sclerosis and pain associated with Parkinson's disease.

Other pain states of visceral origin such as caused by ulcer, dysmenorrhea, endometriosis, irritable bowel syndrome, dyspepsia etc. could also be treated or prevented with Compound **(I)** ethanolamine salt.

A further aspect of the invention is to use Compound **(I)** ethanolamine salt for oral treatment of neuropathic or central pain states.

### Experimental

The invention will now be illustrated by the following non-limiting Examples.

### Example I

### Formation of Solution of Protected Compound (I)

A 1 litre vessel with an overhead stirrer and reflux condenser was set up and inerted with nitrogen. To this was charged water (340 ml), isobutyl [(2-chloropyridin-3-yl)sulfonyl](3-methoxy-5-methylpyrazin-2-yl)carbamate (106 mmol; 48.5 g), [4-(1,3,4-oxadiazol-2-yl) phenyl]boronic acid (Intermediate 2; 164 mmol; 31.8 g), Pd(OAc)₂ (5.85 mmol; 1.31 g), TPPTS [3, 3', 3"-Phospinidynetris(benzenesulfonic acid)trisodium salt] 30% solution in water (17.6 mmol; 28.4 ml; 33.3 g) and isopropyl alcohol (146 ml). The reaction mixture was stirred at 20°C for 12 minutes and then N-methylmorpholine (293 mmol; 32.3 ml; 29.6 g) was added. The reaction mixture was then warmed to 83°C over 90 minutes. After holding at 83°C for 5 hours toluene (340 ml) was added and the reaction mixture was cooled to 60°C and held for 45 mins. The reaction mixture was then filtered through a 1µm filter and the solid washed with toluene (48.5 ml). The filtrates were separated and the undesired aqueous phase discarded. The toluene layer contains a solution of Protected Compound **(I).**

### Example 2

### Formation of Compound (I) using ethanolamine

The above organic layer from Example 1 was adjusted to 42°C and isopropyl alcohol (114 ml), water (170ml) and ethanolamine (28.2 ml) were added and stirred at 42°C for 90 mins. The reaction mixture was allowed to cool to 20°C and the lower aqueous phase separated and filtered through a 1µm filter. The aqueous phase was then charged over 40min to a stirred solution of acetic acid (141 g) and water (33.5 g) at 50°C and then cooled to 20°C over 60 mins. The product was isolated by filtration and washed with a mixture of isopropyl alcohol (48.5 ml) and water (48.5 ml) and then isopropyl alcohol (48.5 ml). The product was dried overnight in a vacuum oven at 55°C. Weight 43.08g, Strength = 100%, 86.7%yield. ¹H NMR (400 MHz, DMSO-d₆) 9.87 (1H, s), 9.14 (1H, s), 8.81 (1H,d), 8.52 (1H, d), 7.98 (2H, d), 7.65 (2H, d), 7.62 (1H, dd), 7.41 (1H, bs), 3.80 (3H, s), 2.23 (3H, s). Mass Spectra MH+ 425.1036 (C₁₉H₁₇N₆O₄S calculated 425.1032).

### Example 3

### Formation of Compound (I) using ammonia

A 150ml flask with an overhead stirrer was set up and inerted with nitrogen. To this was charged Protected Compound **(I)** solution (Example 1; 15.3 mmol; 8.00 g), water (20.0 ml) and industrial methylated spirit (9.20 ml) and the biphasic solution warmed to 60°C. 33% aqueous ammonia solution (137 mmol; 8.05 ml) was added and the reaction mixture held at 60°C for 3 hours. The solution was cooled to 20°C and a premixed solution of acetic acid (14.0 ml) and water (4.56 ml) was added over 17 mins. The product was washed with 1:1 industrial methylated spirit:water (17ml), followed by industrial methylated spirit (9.60 ml). The product was dried overnight in a vacuum oven at 55°C. Weight 5.38g, Strength = 96.7%, yield = 81.2%.

### Example 4

### Compound (I) Ethanolamine Salt

Compound **(I)** (13.5 g at 100%w/w, 30.6 mmol, 1.00 mol equiv.), water (4 ml) and industrial methylated spirit (60 ml) were stirred at 25°C. Ethanolamine (2.0 ml, 2.03 g, 33.2 mmol, 1.09 mol equiv.) was added. The mixture was heated to reflux (79°C), water (0.1 ml) was added and the mixture was maintained at reflux for 15 minutes to give a solution. The stirred solution was allowed to cool to 20°C over 3 hours, during which time a white solid crystallized out. The stirred mixture was cooled to 0 to 1°C over 10 minutes and maintained at this temperature for 30 minutes. The solid was filtered off on a glass sinter under suction and the crystallization flask and the sinter were washed with three successive washes with industrial methylated spirit (3 x 20 ml). The solid was dried on the sinter for 3 hours. Yield = 12.00 g, 81.6%. ¹H NMR (400 MHz, d6 DMSO) 9.36 (1H, s), 8.60-8.61 (1H, dd), 8.36-8.38 (1H, dd), 7.73 (3H, bs), 7.83-7.93 (4H q), 7.42-7.46 (1H, dd), 7.08 (1H, s), 5.07 (1H, bs), 3.64 (3H, s), 3.57 (2H, bt), 2.84 (2H, t), 2.09 (3H, s).

### Reference Example 1

### Compound (I) Sodium Salt

All operations were carried out under an inert atmosphere of nitrogen. Palladium acetate (0.4061 g at 100%w/w, 1.08 mmol, 0.05 mol equiv.) and 3,3',3"-phospinidynetris (benzenesulfonic acid)trisodium salt (3.26 g, 5.56 mmol, 0.15 mol equiv.) were dissolved with agitation in water (35 ml). The resulting yellow solution was added to a stirred slurry of [4-(1,3,4-oxadiazol-2-yl)phenyl]boronic acid (Intermediate 2; 10 g, 52.63 mmol, 1.40 mol equiv.) and isobutyl [(2-chloropyridin-3-yl)sulfonyl](3-methoxy-5-methylpyrazin-2-yl)carbamate (15.65 g, 37.71 mmol, 1.00 mol equiv.) in a mixture of xylene (100 ml), industrial methylated spirit (50 ml) and triethylamine (17 ml, 121.96 mmol, 3.23 mol equiv.). The catalyst make-up vessel was rinsed with water (5 ml) and this solution was added to the reaction mixture. The stirred mixture was heated at reflux (80°C) for 24 hours. The reaction mixture was cooled to 30°C and filtered through a 1 µm glass fibre filter paper under suction. The two-phase filtrates were allowed to settle and the lower aqueous phase was separated. The reaction flask and filter were washed with xylene (20 ml) and these filtrates were used to re-extract the lower aqueous phase. The two phase filtrates were allowed to settle and the lower aqueous phase was separated and discarded. industrial methylated spirit (20 ml) was added to the combined organic phases and the mixture was cooled with stirring to 16°C. Sodium methoxide solution in methanol (11 ml of a 25%w/w solution, 48.13 mmol, 1.28 mol equiv.) was added to the mixture maintaining the temperature at 16 to 19°C. The reaction mixture was stirred at 17 to 19°C for 2 hours. The pH was adjusted to 5- ' 6 by the addition of glacial acetic acid (4.5 ml, 78.54 mmol, 2.08 mol equiv.) to the stirred mixture followed by water (38 ml) at 18 to 26°C. The stirred mixture was heated to 40°C and then allowed to cool to 20°C over 3 hours. The resulting white solid was filtered off on a glass sinter under suction. The flask and the filter were washed with two successive portions of industrial methylated spirit (2 x 25 ml) and the combined filtrates discarded.
The product was dried on the filter for 15 hours. Yield =16.23 g (78.2% theory, correcting for strength). ¹H NMR (400 MHz, d6 DMSO) 9.35 (1H, s), 8.59-8.60 (1H, dd), 8.36-8.38 (1H, dd), 7.86-7.92 (4H q), 7.41-7.45 (1H, dd), 7.08 (1H, s), 3.63 (3H, s), 2.09 (3H, s).

### Reference Example 2

### Compound (I) Ammonium Salt

Compound **(I)** (9.62 g at 100%w/w, 22.67 mmol, 1.00 mol equiv.), industrial methylated spirit (26 ml) and an aqueous solution of ammonia (13 ml of a 35%w/w solution, 235.53 mmol, 10.39 mol equiv.) were stirred at 25°C. The mixture was heated to 50 to 52°C and maintained at this temperature for 15 hours, during which time a white solid crystallized out. Xylene and water were added to the stirred mixture such that the temperature was maintained at 50 to 52°C and this temperature was maintained for 1 hour. The two phase mixture was filtered through a 1µm glass fibre filter paper under suction at 50°C. The dissolution flask and the filter were washed with a solution of industrial methylated spirit (5 ml) and water (5ml) at 50°C. The combined filtrates were stirred at 50 to 52°C for 15 minutes. The two phase solution was allowed to cool to 22°C over 18 hours, during which time a white solid crystallized out. The white solid was filtered on a glass sinter and the crystallization flask and the filter were washed with industrial methylated spirit (13 ml), then water (13 ml) and finally industrial methylated spirit (13 ml) and the filtrates discarded. The white solid was dried on the sinter for 2 hours. Yield = 7.47g (69% of theory corrected for strength). ¹H NMR (400 MHz, d6 DMSO) 9.36 (1H, s), 8.60-8.61 (1H, dd), 8.37-8.39 (1H, dd), 7.82-7.93 (4H q), 7.43-7.46 (1H, dd), 7.13 (4H, bs), 7.08 (1H, s), 3.63 (3H, s), 2.09 (3H, s).

### Reference Example 3

### Compound (I) Ammonium Salt N-methylpyrrolidinone Solvate

Compound **(I)** Ammonium Salt (Reference Example 2; 15.96 g at 100%w/w, 36.16 mmol, 1.00 mol equiv.), was stirred with an aqueous solution of ammonia (19 ml of a 35%w/w solution, 344.24 mmol, 9.52 mol equiv.), water (190 ml) and N-methylpyrrolidinone (70 ml) at 25°C. The mixture was heated to 65°C and maintained at this temperature for 15 minutes to give a solution. The solution was then allowed to cool slowly to 12°C over 15 hours to crystallise the product. The stirred slurry was cooled to 1°C over 19 hours. The solid was filtered off and washed with three successive washes of industrial methylated spirit (3 x 50 ml) and the filtrates were discarded. The solid was air dried on the sinter for 3 hours. Yield = 16.66 g, 83.3%. ¹H NMR (400 MHz, d6 DMSO) 9.35 (1H, s), 8.59-8.60 (1H, dd), 8.36-8.38 (1H, dd), 7.84-7.93 (4H q), 7.42-7.45 (1H, dd), 7.07 (1H, s), 7.06 (4H, bs), 3.63 (3H, s), 2.09 (3H, s).

### Intrinsic Dissolution Rate

The intrinsic dissolution rate of the four salts forms prepared above were compared with Compound **(I)** in pH 6.5 buffer were determined for comparison.

The intrinsic dissolution rate was determined by using a fibre optic UV probe, measuring at 260nm, with a SOTAX dissolution apparatus. Each of the pots in the dissolution batch was filled with 500 ml of pH 6.5 buffer and heated to 37°C. 50 mg Of each compound was weighed out in triplicate. Each of these samples was placed into a 4mm dye and compressed at 50psi for 5 minutes to produce suitable discs. These discs were then placed into the dissolution bath and the UV absorbance measured at regular intervals. Standards were prepared at approximately 20 µg/ml concentrations in pH 6.5 buffer. Scans of the background and corresponding standards were taken and a standard recovery check was produced.

### Results: Intrinsic Dissolution Rates

| **Compound** | **IDR (mg/min/cm²)** |
|---|---|
| Compound **(I)** | 0.05 |
| Compound **(I)** Sodium Salt | 1.37^{a} |
| Compound **(I)** Ethanolamine Salt | 0.84 |
| Compound **(I)** Ammonium Salt | 0.14 |
| Compound **(I)** Ammonium Salt (N-methylpyrrolidinone Solvate) | 0.10 |

| | |
|---|---|
| ^{a} The discs fell apart rapidly during the experiment. Subsequent experiments at higher compression forces to aid disc compaction of the compound did not improve this observation. Therefore the result reported in this case are taken from the initial few minutes of the experiment and should be interpreted as an over estimate of the true IDR of the Compound **(I)** Sodium Salt. | |

### XRPD data on Salts and Polymorphs

The X-ray powder diffraction patterns of Compound **(I)** Ethanolamine Salt, Compound **(I)** Form 1, Compound **(I)** Form 3, Compound **(I)** Sodium Salt, Compound **(I)** Ammonium Salt and Compound **(I)** Ammonium Salt NMP solvate were determined by mounting a sample of the crystalline material on Siemens single silicon crystal (SSC) wafer mounts and spreading out the sample into a thin layer with the aid of a microscope slide. The sample was spun at 30 revolutions per minute (to improve counting statistics) and irradiated with X-rays generated by a copper long-fine focus tube operated at 40 kV and 40 mA with a wavelength of 1.5406 Angstroms using a Bruker D5000 powder X-ray diffractometer (Bruker AXS, Banner Lane Coventry CV4 9GH). The collimated X-ray source was passed through an automatic variable divergence slit set at V20 and the reflected radiation directed through a 2 mm antiscatter slit and a 0.2 mm detector slit. The sample was exposed for 1 second per 0.02 degree 2-theta increment (continuous scan mode) over the range 2 degrees to 40 degrees 2-theta in theta-theta mode. The instrument was equipped with a scintillation counter as detector. Control and data capture was by means of a Dell Optiplex 686 NT 4.0 Workstation operating with Diffract+ software. Data were collected over the range 2-theta 2 - 40°, in increments of 2-theta 0.02° with 4s per increment.

The skilled person is aware that an X-ray powder diffraction pattern may be obtained which has one or more measurement errors depending on measurement conditions (such as equipment, sample preparation or machine used). In particular, it is generally known that intensities in an X-ray powder diffraction pattern may fluctuate depending on measurement conditions and sample preparation. For example, the skilled person will realize that the relative intensity of peaks can be affected by, for example, grains above 30 microns in size and non-unitary aspect ratios, which may affect analysis of samples. The skilled person will also realize that the position of reflections can be affected by the precise height at which the sample sits in the diffractometer and the zero calibration of the diffractometer. The surface planarity of the sample may also have a small effect. Hence a person skilled in the art will appreciate that the diffraction pattern data presented herein is not to be construed as absolute (for further information see Jenkins, R & Snyder, R.L. 'Introduction to X-Ray Powder Diffractometry' John Wiley & Sons, 1996). Therefore, it shall be understood that the crystalline form of Compound **(I)** Ethanolamine Salt, Compound **(I)** Form 1, Compound **(I)** Form 3, Compound **(I)** Sodium Salt, Compound **(I)** Ammonium Salt and Compound **(I)** Ammonium Salt NMP solvate is not limited to the crystals that provide X-ray powder diffraction patterns identical to the X-ray powder diffraction patterns shown in Figures 1 to 6 and any crystals providing X-ray powder diffraction patterns substantially the same as that shown in Figures 1 to 6 fall within the scope of the present invention. A person skilled in the art of X-ray powder diffraction is able to judge the substantial identity of X-ray powder diffraction patterns.

### XRPD of Compound (I) Form 3

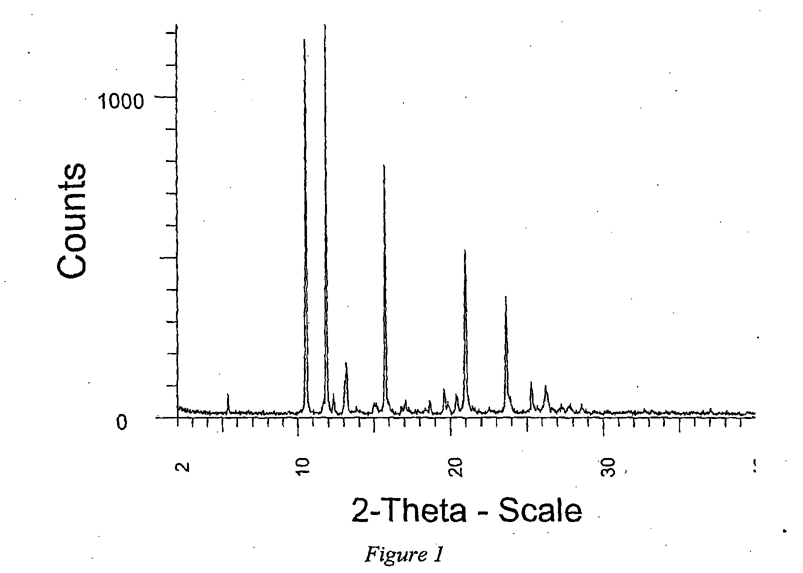

**Table 1: Distinguishing peaks plus most intense peaks (in order of intensity) for Compound (I) Form 3**

| **Angle 2-Theta (2**θ**)** | **Relative Intensity** |
|---|---|
| 11.8 | VS |
| 10.5 | VS |
| 15.7 | VS |
| 21.0 | VS |
| 23.6 | VS |
| 13.1 | S |
| 25.3 | M |
| 26.3 | M |
| 19.6 | M |
| 5.3 | M |

### XRPD of Compound (I) Sodium salt

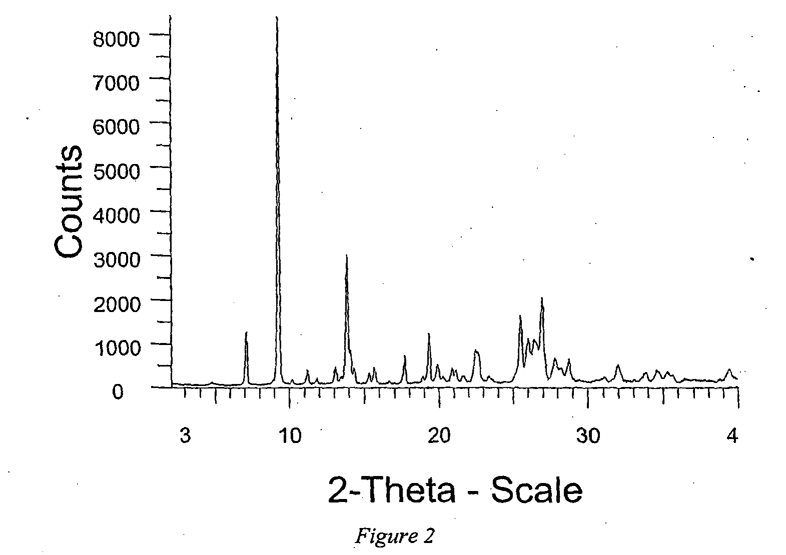

**Table 2: Distinguishing peaks plus most intense peaks (in order of intensity) for Compound (I) Sodium salt**

| **Angle 2-Theta (2**θ**)** | **Relative Intensity** |
|---|---|
| 9.3 | VS |
| 13.8 | VS |
| 26.9 | S |
| 25.5 | S |
| 7.1 | S |
| 19.3 | S |
| 26.1 | S |
| 26.5 | S |
| 22.5 | M |
| 17.7 | M |

### XRPD of Compound (I) Ethanolamine Salt

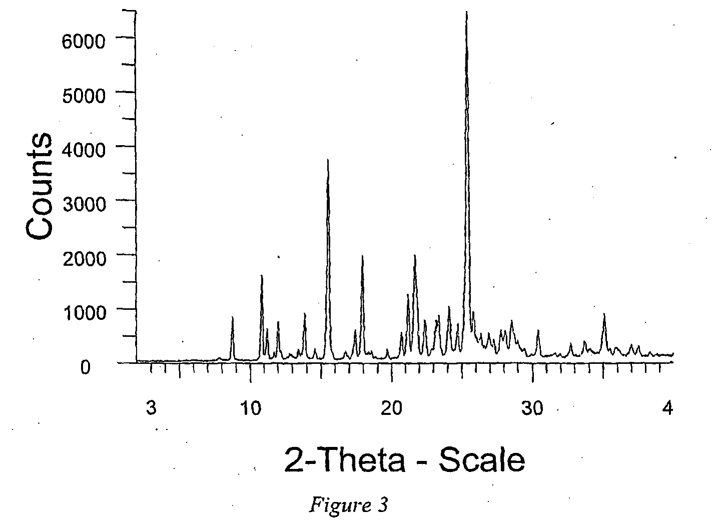

**Table 3: Distinguishing peaks plus most intense peaks (in order of intensity) for Compound (I) Ethanolamine Salt**

| **Angle 2-Theta (2**θ**)** | **Relative Intensity** |
|---|---|
| 25.5 | VS |
| 15.5 | VS |
| 21.7 | VS |
| 18.0 | VS |
| 10.9 | S |
| 21.2 | S |
| 24.1 | S |
| 25.9 | S |
| 13.9 | S |
| 35.2 | S |
| 8.9 | M |

### XRPD of Compound (I) Ammonium Salt

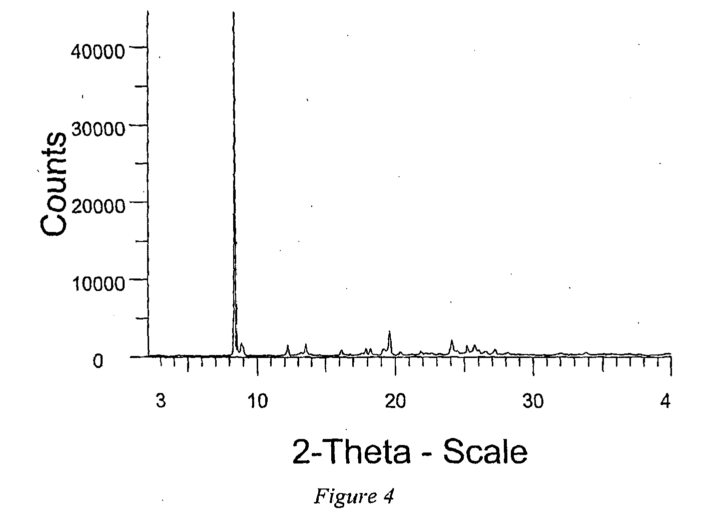

**Table 4: Distinguishing peaks plus most intense peaks (in order of intensity) for Compound (I) Ammonium Salt**

| **Angle 2-Theta (2**θ**)** | **Relative Intensity** |
|---|---|
| 8.4 | VS |
| 19.6 | M |
| 24.1 | M |
| 8.8 | M |
| 13.5 | M |
| 25.7 | M |
| 12.2 | M |
| 25.2 | M |
| 17.8 | W |
| 18.2 | W |

### XRPD of Compound (I) Ammonium salt NMP solvate

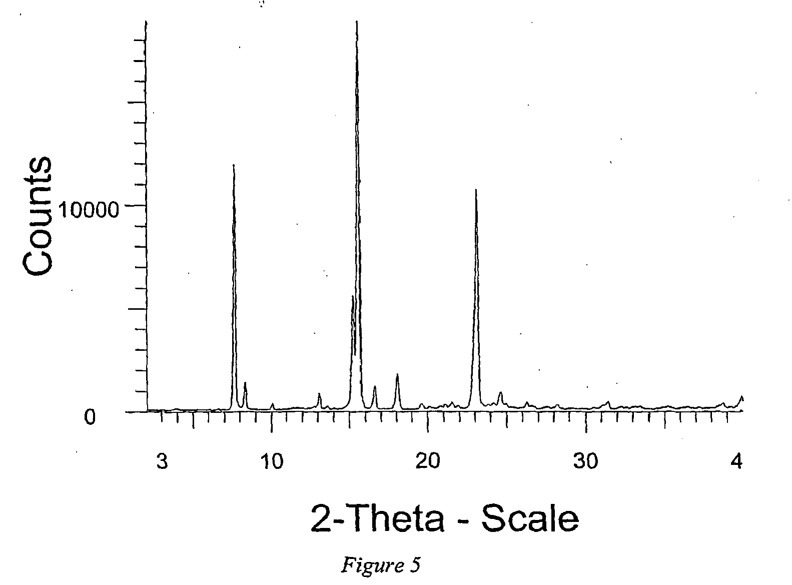

**Table 5: Distinguishing peaks plus most intense peaks (in order of intensity) for Compound (I) Ammonium salt NMP solvate**

| **Angle 2-Theta (2**θ**)** | **Relative Intensity** |
|---|---|
| 15.6 | VS |
| 7.6 | VS |
| 23.1 | VS |
| 15.2 | VS |
| 18.0 | M |
| 8.3 | M |
| 16.6 | M |
| 24.6 | M |
| 13.0 | M |
| 31.4 | W |
| 10.0 | W |

### XRPD of Compound (I) Form 1

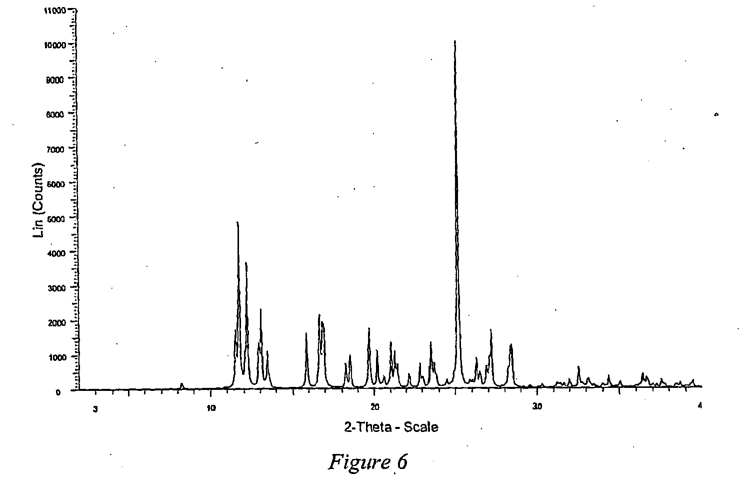

**Table 6: Distinguishing peaks plus most intense peaks (in order of intensity) for Compound (I) Form 1**

| **Angle 2-Theta (2**θ**)** | **Relative Intensity** |
|---|---|
| 25.2 | VS |
| 11.7 | VS |
| 12.2 | S |
| 13.0 | S |
| 16.6 | M |
| 16.8 | M |
| 16.9 | M |
| 19.7 | M |
| 27.2 | M |
| 11.5 | M |

### Intermediates

### Intermediate 1

### 2-(4-Bromophenyl)-1,3,4-oxadiazole

To a suspension of 4-bromobenzoie hydrazide (200 g) in industrial methylated spirit (700 ml) was added triethylorthoformate (309 ml), industrial methylated spirit (100 ml) and sulphuric acid (0.8 ml). The reaction mixture was heated to reflux for 1 hour. The reaction mixture was cooled to 0-5°C and product crystallised. Product was isolated, washed and dried to yield 2-(4-bromophenyl)-1,3,4-oxadiazole (186.1 g, 89.9%). 400MHz NMR Spectrum: (DMSOd₆) 9.35 (s, 1H), 7.98 (d, 1H), 7.95 (d, 1H), 7.84 (d, 1H), 7.81 (d, 1H); Mass Spectrum MH⁺ 224.9663 (calc. using 79-Br) Found 224.9701.

### Intermediate 2

### [4-(1,3,4-Oxadiazol-2-yl)phenyl]boronic acid

A solution of methyllithium (8% w/w in diethoxymethane) (65 ml) was added to a suspension of 2-(4-bromophenyl)-1,3,4-oxadiazole (Intermediate 1; 40 g) in tetrahydrofuran (THF) (415 ml) at -65°C. After an hour a solution of n-butyllithium (2.5M in hexanes) (78 ml) was then added at -65°C. After an hour, triisopropylborate (90 ml)) was then added maintaining the reaction mixture at -65°C. The reaction mixture was held at -65°C for an hour and then warmed to -20°C and drowned out into a mixture of acetic acid (28 ml) in water (222 ml). The resultant solid was isolated, washed with THF and water, and dried to yield the title compound (28.96 g @ 95.1% w/w, 82%); 400MHz NMR Spectrum: (DMSOd₆) 8.00 (s, 4H), 8.31 (s, 2H), 9.35 (s, 1H); Mass Spectrum MR⁺ 191.0628 (calc. using 11-B) Found 191.0633.

## Claims

1. *N*-(3-Methoxy-5-methylpyrazin-2-yl)-2-(4-[1,3,4-oxadiazol-2-yl]phenyl)pyridine-3-sulphonamide ethanolamine salt.

2. The salt according to claim 1, further **characterized in that** the compound is in substantially crystalline form.

3. The salt according to claim 2, further **characterized in that** the compound has an X-ray powder diffraction pattern containing at least peaks with 2-theta values at 8.9°, 10.9° and 18° measured using CuKa radiation.

4. The salt according to claim 3, further **characterized in that** the compound has an X-ray powder diffraction pattern containing at least peaks with 2-theta values at 8,9°, 10.9°, 18°, 25.5°, 15.5° and 21.7° measured using CuKa radiation,

5. The salt according to claim 4, further **characterized in that** the compound has an X-ray powder diffraction pattern containing at least peaks with 2-theta values at 8.9°, 10.9°, 18°, 25.5°, 15.5°, 21.7, 21.2°, 24.1° and 25.9° measured using CuKa radiation.

6. A compound according to claim 5, **characterized by** an X ray diffraction pattern essentially as defined in Figure 3 and/or in Table 3:
**Table 3**
| **Angle 2-Theta (2**θ**)** | **Relative Intensity** |
|---|---|
| 25.5 | VS |
| 15.5 | VS |
| 21.7 | VS |
| 18.0 | VS |
| 10.9 | S |
| 21.2 | S |
| 24.1 | S |
| 25.9 | S |
| 13.9 | S |
| 35.2 | S |
| 8.9 | M |

7. A process for the preparation of *N*-(3-methoxy-5-methylpyrazin-2-yl)-2-(4-[1,3,4-oxadiazol-2-yl]phenyl)pyridine-3-sulphonamide which comprises the use of ethanolamine to deprotect a compound of formula **(II)**: 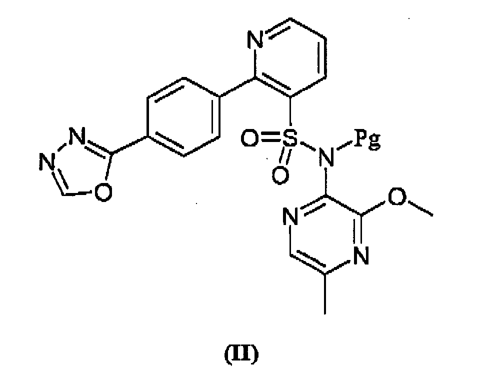 where Pg is a suitable nitrogen protecting group.

8. The process according to claim 7 wherein Pg is isobutoxycarbonyl.

9. A process for the manufacture of *N*-(3-methoxy-5-methylpyrazin-2-yl)-2-(4-[1,3,4-oxadiazol-2-yl]phenyl)pyridine-3-sulphonamide substantially in the form of Form 1 which comprises:
(i) the use of ethanolamine to deprotect a compound of formula (II): 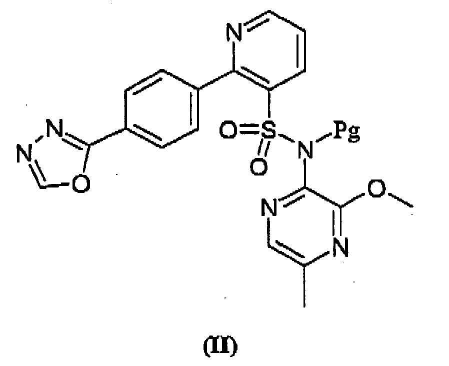
where Pg is a suitable nitrogen protecting group; followed by (ii) the addition of the resulting *N*-(3-methoxy-5-methylpyrazin-2-yl)-2-(4-[1,3,4-oxadiazol-2-yl]phenyl)pyridine-3-sulphonamide ethanolamine salt to an acid.

10. The process according to claim 9 wherein Pg is isobutoxycarbonyl.

11. The process according to claim 9 or claim 10 wherein the acid is acetic acid.

12. The use of the salt according to claim 1 in the preparation of *N*-(3-methoxy-5-methylpyrazin-2-yl)-2-(4-[1,3,4-oxadiazol-2-yl]phenyl)pyridine-3-sulphonamide.

13. The use of the salt according to claim 1 in the manufacture of *N*-(3-methoxy-5-methylpyrazin-2-yl)-2-(4-[1,3,4-oxadiazol-2-yl]phenyl)pyridine-3-sulphonamide substantially in the form of Form 1.

14. A pharmaceutical composition which comprises the salt according to any one of claims 1-6 in association with a pharmaceutically acceptable diluent or carrier.

15. The salt according to any one of claims 1-6 for use as a medicament.

16. The use of the salt according to any one of claims 1-6, in the manufacture of a medicament for the treatment of cancer in a warm blooded animal such as man.

17. The use according to claim 16 wherein the cancer is oesophageal cancer, myeloma, hepatocellular, pancreatic, cervical cancer, ewings tumour, neuroblastoma, Kaposis sarcoma, ovarian cancer, breast cancer, colorectal cancer, prostate cancer, bladder cancer, melanoma, lung cancer - non small cell lung cancer, and small cell lung cancer, gastric cancer, head and neck cancer, renal cancer lymphoma and leukaemia.

18. The use according to claim 16 wherein the cancer is prostate cancer.

19. The use according to any one of claims 16-18 wherein the cancer is in a metastatic state.

20. The use according to any one of claims 16-18 wherein the cancer is in a non-metastatic state.

21. The use according to claim 16 wherein the cancer is renal, thyroid, lung, breast or prostate cancer that is producing bone metastases.

## Patentansprüche

1. *N*-(3-Methoxy-5-methylpyrazin-2-yl)-2-(4-[1,3,4-oxadiazol-2-yl]phenyl)pyridin-3-sulfonamid-Ethanolamin-Salz.

2. Salz nach Anspruch 1, ferner **dadurch gekennzeichnet, daß** die Verbindung in weitgehend kristalliner Form vorliegt.

3. Salz nach Anspruch 2, ferner **dadurch gekennzeichnet, daß** die Verbindung ein Röntgenpulverbeugungsmuster aufweist, das mindestens Peaks mit 2-Theta-Werten bei 8,9°, 10,9° und 18°, gemessen unter Verwendung von CuKa-Strahlung, enthält.

4. Salz nach Anspruch 3, ferner **dadurch gekennzeichnet, daß** die Verbindung ein Röntgenpulverbeugungsmuster aufweist, das mindestens Peaks mit 2-Theta-Werten bei 8,9°, 10,9°, 18°, 25,5°, 15,5° und 21,7°, gemessen unter Verwendung von CuKa-Strahlung, enthält.

5. Salz nach Anspruch 4, ferner **dadurch gekennzeichnet, daß** die Verbindung ein Röntgenpulverbeugungsmuster aufweist, das mindestens Peaks mit 2-Theta-Werten bei 8,9°, 10,9°, 18°, 25,5°, 15,5°, 21,7°, 21,2°, 24,1° und 25,9°, gemessen unter Verwendung von CuKa-Strahlung, enthält.

6. Verbindung nach Anspruch 5, **gekennzeichnet durch** ein Röntgenbeugungsmuster, das im wesentlichen wie in Figur 3 und/oder in Tabelle 3 definiert ist:
**Tabelle 3**
| **2-Theta-Winkel (2**θ**)** | **Relative Intensität** |
|---|---|
| 25,5 | VS |
| 15,5 | VS |
| 21,7 | VS |
| 18,0 | VS |
| 10,9 | S |
| 21,2 | S |
| 24,1 | S |
| 25,9 | S |
| 13,9 | S |
| 35,2 | S |
| 8,9 | M |

7. Verfahren zur Herstellung von *N*-(3-Methoxy-5-methylpyrazin-2-yl)-2-(4-[1,3,4-oxadiazol-2-yl]-phenyl)pyridin-3-sulfonamid, bei dem man zur Entschützung einer Verbindung der Formel (II): 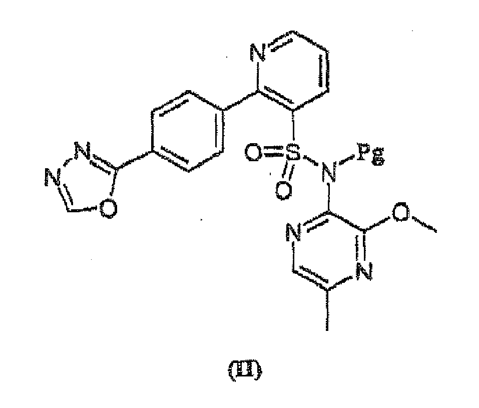 worin Pg für eine geeignete Stickstoff-Schutzgruppe steht, Ethanolamin verwendet.

8. Verfahren nach Anspruch 7, bei dem Pg für Isobutoxycarbonyl steht.

9. Verfahren zur Herstellung von weitgehend in Form von Form 1 vorliegendem *N*-(3-Methoxy-5-methylpyrazin-2-yl)-2-(4-[1,3,4-oxadiazol-2-yl]phenyl)-pyridin-3-sulfonamid, bei dem man:
(i) zur Entschützung einer Verbindung der Formel (II): 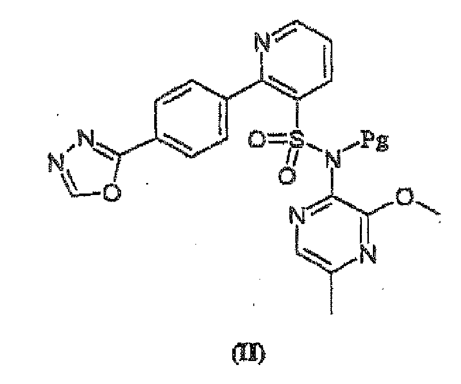
worin Pg für eine geeignete Stickstoff-Schutzgruppe steht, Ethanolamin verwendet und dann (ii) das resultierende *N*-(3-Methoxy-5-methylpyrazin-2-yl)-2-(4-[1,3,4-oxadiazol-2-yl]phenyl)-pyridin-3-sulfonamid-Ethanolamin-Salz zu einer Säure gibt.

10. Verfahren nach Anspruch 9, bei dem Pg für Isobutoxycarbonyl steht.

11. Verfahren nach Anspruch 9 oder Anspruch 10, bei dem es sich bei der Säure um Essigsäure handelt.

12. Verwendung des Salzes nach Anspruch 1 bei der Herstellung von *N*-(3-Methoxy-5-methylpyrazin-2-yl)-2-(4-[1,3,4-oxadiazol-2-yl]phenyl)pyridin-3-sulfonamid.

13. Verwendung des Salzes nach Anspruch 1 bei der Herstellung von weitgehend in Form von Form 1 vorliegendem *N*-(3-Methoxy-5-methylpyrazin-2-yl)-2-(4-[1,3,4-oxadiazol-2-yl]phenyl)pyridin-3-sulfonamid.

14. Pharmazeutische Zusammensetzung, die das Salz nach einem der Ansprüche 1-6 zusammen mit einem pharmazeutisch annehmbaren Verdünnungsmittel oder Träger enthält.

15. Salz nach einem der Ansprüche 1-6 zur Verwendung als Arzneimittel.

16. Verwendung des Salzes nach einem der Ansprüche 1-6 bei der Herstellung eines Arzneimittels zur Verwendung bei der Behandlung von Krebs bei einem Warmblüter wie dem Menschen.

17. Verwendung nach Anspruch 16, bei der es sich bei dem Krebs um Speiseröhrenkrebs, Myelom, Leberzellkrebs, Bauchspeicheldrüsenkrebs, Zervixkrebs, Ewing-Sarkom, Neuroblastom, Kaposi-Sarkom, Eierstockkrebs, Brustkrebs, Kolorektalkrebs, Prostatakrebs, Blasenkrebs, Melanom, Lungenkrebs - nicht-kleinzelligen Lungenkrebs und kleinzelligen Lungenkrebs, Magenkrebs, Kopf- und Halskrebs, Nierenkrebs, Lymphom und Leukämie handelt.

18. Verwendung nach Anspruch 16, bei der es sich bei dem Krebs um Prostatakrebs handelt.

19. Verwendung nach einem der Ansprüche 16-18, bei der sich der Krebs in einem metastasierenden Stadium befindet.

20. Verwendung nach einem der Ansprüche 16-18, bei der sich der Krebs in einem nicht metastasierenden Stadium befindet.

21. Verwendung nach Anspruch 16, bei der es sich bei dem Krebs um Knochenmetastasen produzierenden Nieren-, Schilddrüsen-, Lungen-, Brust- oder Prostatakrebs handelt.

## Revendications

1. Sel de l'éthanolamine de *N*-(3-méthoxy-5-méthylpyrazin-2-yl)-2-(4-[1,3,4-oxadiazol-2-yl]phényl)pyridine-3-sulfonamide.

2. Sel selon la revendication 1, **caractérisé en outre en ce que** le composé est sous une forme sensiblement cristalline.

3. Sel selon la revendication 2, **caractérisé en outre en ce que** le composé a un diagramme de diffraction des rayons X sur poudre contenant au moins des pics ayant des valeurs 2-thêta à 8,9°, 10,9° et 18° mesurées en utilisant des radiations de CuKα.

4. Sel selon la revendication 3, **caractérisé en outre en ce que** le composé a un diagramme de diffraction des rayons X sur poudre contenant au moins des pics ayant des valeurs 2-thêta à 8,9°, 10,9°, 18°, 25,5°, 15,5° et 21,7° mesurées en utilisant des radiations de CuKα.

5. Sel selon la revendication 4, **caractérisé en outre en ce que** le composé a un diagramme de diffraction des rayons X sur poudre contenant au moins des pics ayant des valeurs 2-thêta à 8,9°, 10,9°, 18°, 25,5°, 15,5°, 21,7°, 21,2°, 24,1° et 25,9° mesurées en utilisant des radiations de CuKα.

6. Composé selon la revendication 5, **caractérisé par** un diagramme de diffraction des rayons X essentiellement tels que définis à la Figure 3 et/ou dans le Tableau 3 :
**Tableau 3**
| **Angle 2-thêta (2**θ**)** | **Intensité relative** |
|---|---|
| 25,5 | VS |
| 15,5 | VS |
| 21,7 | VS |
| 18,0 | VS |
| 10,9 | S |
| 21,2 | S |
| 24,1 | S |
| 25,9 | S |
| 13,9 | S |
| 35,2 | S |
| 8,9 | M |

7. Procédé de préparation de *N*-(3-méthoxy-5-méthylpyrazin-2-yl)-2-(4-[1,3,4-oxadiazol-2-yl]phényl)pyridine-3-sulfonamide qui comprend l'utilisation de l'éthanolamine pour déprotéger un composé de formule (II) : 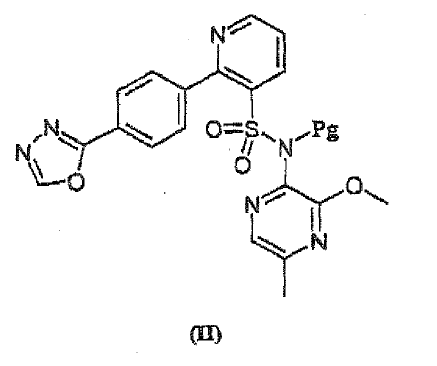 dans laquelle Pg est un groupement protecteur d'azote convenable.

8. Procédé selon la revendication 7, **caractérisé en ce que** Pg est isobutoxycarbonyle.

9. Procédé de fabrication de *N*-(3-méthoxy-5-méthylpyrazin-2-yl)-2-(4-[1,3,4-oxadiazol-2-yl]phényl)pyridine-3-sulfonamide sensiblement sous forme de la Forme 1 qui comprend :
(i) l'utilisation de l'éthanolamine pour déprotéger un composé de formule (II) : 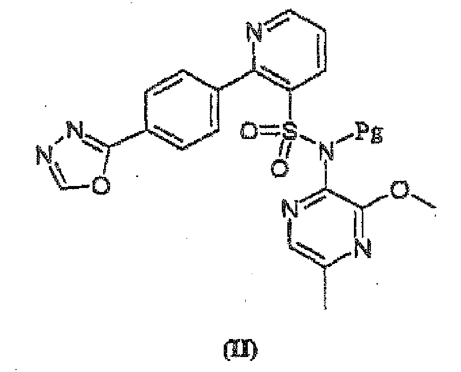 dans laquelle Pg est un groupement protecteur d'azote convenable ; suivie de
(ii) l'addition du sel de l'éthanolamine de *N*-(3-méthoxy-5-méthylpyrazin-2-yl)-2-(4-[1,3,4-oxadiazol-2-yl]phényl)pyridine-3-sulfonamide résultant à un acide.

10. Procédé selon la revendication 9, **caractérisé en ce que** Pg est isobutoxycarbonyle.

11. Procédé selon la revendication 9 ou la revendication 10, **caractérisé en ce que** l'acide est l'acide acétique.

12. Utilisation du sel selon la revendication 1, dans la préparation du *N*-(3-méthoxy-5-méthylpyrazin-2-yl)-2-(4-[1,3,4-oxadiazol-2-yl]phényl)pyridine-3-sulfonamide.

13. Utilisation du sel selon la revendication 1, dans la fabrication du *N*-(3-méthoxy-5-méthylpyrazin-2-yl)-2-(4-[1,3,4-oxadiazol-2-yl]phényl)pyridine-3-sulfonamide sensiblement sous forme de la Forme 1.

14. Composition pharmaceutique qui comprend le sel selon l'une quelconque des revendications 1-6, en association avec un diluant ou un support pharmaceutiquement acceptable.

15. Sel selon l'une quelconque des revendications 1-6, destiné à être utilisé comme médicament.

16. Utilisation du sel selon l'une quelconque des revendications 1-6, dans la fabrication d'un médicament destiné au traitement du cancer chez un animal à sang chaud tel que l'homme.

17. Utilisation selon la revendication 16, **caractérisée en ce que** le cancer est le cancer de l'oesophage, un myélome, le cancer hépatocellulaire, pancréatique, du col de l'utérus, une tumeur d'Ewing, un neuroblastome, le sarcome de Kaposi, le cancer de l'ovaire, le cancer du sein, le cancer colorectal, le cancer de la prostate, le cancer de la vessie, un mélanome, le cancer du poumon - le cancer du poumon non à petites cellules, et le cancer du poumon à petites cellules, le cancer gastrique, le cancer de la tête et du cou, le cancer du rein, un lymphome ou une leucémie.

18. Utilisation selon la revendication 16, **caractérisée en ce que** le cancer est le cancer de la prostate.

19. Utilisation selon l'une quelconque des revendications 16-18, **caractérisée en ce que** le cancer est à un stade métastatique.

20. Utilisation selon l'une quelconque des revendications 16-18, **caractérisée en ce que** le cancer est à un stade non métastatique.

21. Utilisation selon la revendication 16, **caractérisée en ce que** le cancer est le cancer du rein, de la thyroïde, du poumon, du sein ou de la prostate qui produit des métastases osseuses.
